# EUROPEAN PATENT SPECIFICATION

(11) **EP 3 797 295 B1**
(45) Date of publication and mention of the grant of the patent: **16.08.2023**
(21) Application number: 19727472.3
(22) Date of filing: 23.05.2019
(51) Int. Cl.: G01N 33/574

(54) **ISOPROPANOL AS A BIOMARKER FOR COLORECTAL CANCER**
ISOPROPANOL ALS BIOMARKER FÜR KOLOREKTALKARZINOM
L'ISOPROPANOL COMME BIOMARQUEUR DU CANCER COLORECTAL

(30) Priority: 23.05.2018 GB 201808476
(43) Date of publication of application: 31.03.2021
(73) Proprietor: The University of Liverpool, Liverpool, Merseyside L69 7ZX (GB); University Hospitals Bristol NHS Foundation Trust, Bristol BS1 3NU (GB)
(72) Inventor: PROBERT, Chris, Liverpool Merseyside L69 7ZX (GB); BOND, Ashley, Liverpool Merseyside L69 7ZX (GB); GREENWOOD, Rosemary, Bristo Bristol BS1 3NU (GB)
(74) Representative: HGF
(86) International application number: PCT/GB2019/051421
(87) International publication number: WO 2019/224542

(56) References cited:
- CLAIRE A BATTY ET AL: "Use of the Analysis of the Volatile Faecal Metabolome in Screening for Colorectal Cancer", PLOS ONE, vol. 10, no. 6, 18 June 2015 (2015-06-18), page e0130301, XP55605787, DOI: 10.1371/journal.pone.0130301
- WEHINGER ET AL: "Lung cancer detection by proton transfer reaction mass-spectrometric analysis of human breath gas", INTERNATIONAL JOURNAL OF MASS SPECTROMETRY, ELSEVIER SCIENCE PUBLISHERS, AMSTERDAM, NL, vol. 265, no. 1, 4 July 2007 (2007-07-04), pages 49-59, XP022142662, ISSN: 1387-3806, DOI: 10.1016/J.IJMS.2007.05.012
- I. AHMED ET AL: "Investigation of faecal volatile organic metabolites as novel diagnostic biomarkers in inflammatory bowel disease", ALIMENTARY PHARMACOLOGY & THERAPEUTICS., vol. 43, no. 5, 25 January 2016 (2016-01-25), pages 596-611, XP055272092, GB ISSN: 0269-2813, DOI: 10.1111/apt.13522
- ASHLEY BOND ET AL: "Volatile organic compounds emitted from faeces as a biomarker for colorectal cancer", ALIMENTARY PHARMACOLOGY & THERAPEUTICS., vol. 49, no. 8, 3 March 2019 (2019-03-03), pages 1005-1012, XP55605797, GB ISSN: 0269-2813, DOI: 10.1111/apt.15140

## Description

### INTRODUCTION

The present invention relates to certain volatile organic compounds that can be used as biomarkers for colorectal cancer. More specifically, the present invention relates to a method of determining the probability that an individual has colorectal cancer by detecting the presence and amount of certain volatile organic compounds in a stool sample collected from the individual concerned.

### BACKGROUND OF THE INVENTION

Colorectal cancer is a leading cause of mortality and morbidity worldwide, with an estimated European incidence of 43.5 per 100,000 in 2012 and mortality of 19.5 per 100,000.^{[4]} The lifetime risk, for UK residents, is 1 in 15 for men or 1 in 19 for women.^{[5]} Across Europe, colorectal cancer is the second most common cause of cancer related mortality.^{[4]} Colorectal cancer carries a significant financial burden for the National Health Service (NHS), with a mean annual cost of £12,000 and £8,800 for each patient diagnosed with rectal and non-rectal colon cancer, respectively.^{[6]} Data from the UK Bowel Cancer Screening Programme (UK BCSP) has clearly demonstrated that detection of colorectal cancer at an earlier stage, and identification of advanced pre-malignant adenomas, can reduce future cancer-associated mortality and morbidity.^{[7,8]}

The UK BCSP uses a faeces-based screening tool to select patients to take forward to colonoscopy, in line with European guidance.^{[9]} Currently, in England, the guaiac-based faecal occult blood testing (gFOBt) is employed. This test relies upon bleeding from neoplastic lesions and can be used to identify people with >10ml blood loss daily. gFOBt is prone to false positive results after ingestion of certain foods.^{[10]} The low sensitivity of gFOBt has led to criticism of its use for population-based screening.^{[11]} The gFOBt is likely to be replaced by immunological FOBT which can provide both qualitative and quantitative results. A recent observational study, from Italy, demonstrated a reduction in CRC-related mortality in regions where screening with this new approach was adopted compared with regions where screening had not yet been implemented.^{[12,13]} In 2010, a meta-analysis reported a sensitivity (67 %) and specificity (85 %) for immunological FOBt compared with 54 % and 80 % for gFOBt, for the detection of colorectal cancer and pre-malignant neoplasia.^{[14]}

Several studies have suggested volatile organic compounds (VOCs) emitted from different substrates may be used as biomarkers for colorectal cancer. One such study used selected ion flow tube mass spectrometry (SIFT-MS) to detect VOCs in faeces.^{[15]} Another analysed urine, from patients with colorectal cancer, employing Field Asymmetric Ion Mobility Spectrometer (FAIMS).^{[16]} A third used breath analysed by thermal-desorber gas chromatography-mass spectrometry in an attempt to diagnose colorectal cancer.^{[17,18]} However, none of these studies identified specific volatile organic compounds that could be detected, quantified and used as specific biomarkers for colorectal cancer. Instead, these studies were predominately proof of concept or feasibility studies that reported output patterns and general trends only.

Therefore, there remains a need for need for new and improved methods for determining the probability that an individual has colorectal cancer that is preferably both quick and non-invasive.

The present invention was devised with the forgoing in mind.

### SUMMARY OF THE INVENTION

The present invention resides in the recognition that certain volatile organic compounds (VOCs) present in the faeces of an individual can be used as biomarkers for colorectal cancer (i.e. cancer of the colon and/or rectum).

The faeces of an individual contains numerous volatile molecules, which are byproducts of various metabolic pathways in the body. Controlled measurement of these excreted compounds has identified particular VOC biomarkers that are associated with colorectal cancer. These biomarkers provide novel phenotype-specific biomarkers.

The present invention therefore provides methodology for determining the probability that an individual has colorectal cancer by detecting and quantifying the amounts of certain volatile organic compounds that are present in an excreted stool sample and using these compounds as biomarkers for colorectal cancer.

Thus, in one aspect, the present invention provides a method of determining the probability that an individual has colorectal cancer, the method comprising the steps of:
i) analysing a stool sample collected from said individual to detect whether the volatile organic compound isopropanol is present;
ii) if isopropanol is present in step i), determining the amount of isopropanol that is present and comparing the amount detected with a control value;
wherein the presence of an increased amount of isopropanol in the stool sample compared to the control value is indicative of an increased probability that the individual has colorectal cancer.

Also disclosed but not claimed is a method of determining the probability that an individual has colorectal cancer by analysing one or more stool samples collected from the individual to determine the amount of isopropanol that is present, and comparing the amount detected with a control value.

In another aspect, the present invention provides a use of isopropanol in a stool sample as a biomarker for colorectal cancer.

In yet another aspect, the present invention provides a use of isopropanol and one or more additional volatile organic compounds in a stool sample as biomarkers for colorectal cancer, wherein the one or more additional volatile organic compounds are selected from:
i) 2-hexanone;
ii) Butanoic acid, 3-methyl-, ethyl ester;
iii) Butanoic acid, 1-methylethyl ester;
iv) Pentanoic acid, 1-methylethyl ester;
v) p-Xylene;
vi) Propanoic acid, 1-methylethyl ester; or
vii) Dimenthol.

It will be appreciated that the methods of the present invention are conducted outside of the human or animal body and are instead conducted on a stool sample that is collected from the individual concerned. Accordingly, the methodology of the present invention provides a non-invasive means of determining the probability that an individual has colorectal cancer.

### DETAILED DESCRIPTION OF THE INVENTION

### Definitions

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The term "colorectal cancer" used herein will be understood to refer to cancer associated with any part of the colon and/or rectum.

The term "stool sample" used herein refers to a sample of faecal matter obtained from an individual.

The term "volatile organic compound" or "VOC" used herein will be understood as referring to an organic compound that has a high vapour pressure (volatility) at ambient temperature and pressure. Volatile organic compounds typically have a boiling point of less than or equal to 250 °C when measured at a standard atmospheric pressure of 101.3 kPa (1 atmosphere).

The term "control value" used herein will be understood as referring to the average or normalised amount (e.g. concentration) of a given volatile organic compound that is present in a stool sample taken from a control group. In this regard, it will be understood that step ii) of the present invention involves determining the relative amount of isopropanol that is present in a stool sample of an individual compared to the average or normalised amount of isopropanol detected in the stool sample of the control group. It will also be understood that in certain instances, the average or normalised amount (e.g. concentration) of a given volatile organic compound (e.g. isopropanol) that is present in a stool sample taken from a control group may be zero (i.e. it is possible that no isopropanol is present in the stool sample taken from a control group). In such situations, it will be appreciated that the present method will therefore only comprise step i) (i.e. detecting for the presence, or absence, of the volatile organic compound isopropanol in the stool sample of the individual). Furthermore, the "control group" comprises a cohort of individuals who do not have colorectal cancer. Suitably, the "control group" is made up of a cohort of patients who have displayed one or more colorectal symptoms (i.e. symptoms commonly associated with, or possibly associated with, a colorectal disease, including but not limited to cancer) but, following a colonoscopy, a CT colonography, an MRI colonography and/or a barium enema were subsequently determined to be cancer free. For example, terminology such as "comparing the amount of isopropanol present in step i) with a *control value"* used herein, will be readily understood as referring to the comparison between the relative amount of isopropanol present in step i) of the method defined herein, and an average or normalised amount (e.g. concentration) of isopropanol that is present in a stool sample taken from the control group. The size and demographic of the control group may of course vary. Further details of specific control groups that may be used are provided in the examples section hereinbelow.

The chemical names assigned to the chemical compounds (VOCs) herein are names that will be recognised by those skilled in the art. It is appreciated that alternative conventions exist for naming chemical compounds, but a person skilled in the art will appreciate, for example, that "butanoic acid, 3-methyl-, ethyl ester" is a compound that could be alternatively named as "ethyl 3-methylbutanoate", "ethyl 3-methylbutyrate" or "3-methylbutanoic acid ethyl ester". Similarly, ethanol may also be referred to as, for example, ethyl alcohol. The present specification should be interpreted with this in mind.

### Methods of the present invention

As defined hereinabove, according to one aspect of the present invention there is provided a method of determining the probability that an individual has colorectal cancer, the method comprising the steps of:
i) analysing a stool sample collected from said individual to detect whether the volatile organic compound isopropanol is present;
ii) if isopropanol is present in step i), determining the amount of isopropanol that is present and comparing the amount with a control value;
wherein the presence of an increased amount of isopropanol in the stool compared to the control value is indicative of an increased probability that the individual has colorectal cancer.

An increase in the amount of isopropanol present relative to a control value (obtained from a cohort of cancer free individuals), indicates an increased probability that the individual has colorectal cancer and further diagnostic assessment, e.g. by undertaking a colonoscopy, is therefore warranted. Suitably, the increase in the amount of isopropanol relative to the control value is a statistically significant increase (e.g. with a Student's t-Test p value of <0.01, <0.001 or <0.0001).

It will be appreciated that the stool sample may be analysed using any conventional technique known in the art that is capable of detecting the presence of specific compounds from within a sample (e.g. chromatography).

In an embodiment, gas chromatography mass spectrometry (GC-MS) or liquid chromatography is used to analyse and detect the amount of volatile organic compound(s) in the stool sample. Suitably, gas chromatography mass spectrometry (GC-MS) is used to analyse and detect the amount of volatile organic compound(s) in the stool sample.

In another embodiment, the amount of volatile organic compound(s) in the stool sample of the individual is analysed and detected using an antibody-based test. Non-limiting examples of suitable antibody-based tests include enzyme-linked immunosorbent assays (ELISA) or dipstick assays.

In a particular embodiment of the present invention, step i) of the method further comprises analysing the stool sample collected from said individual to detect for the presence of one or more additional volatile organic compounds and, if one or more additional volatile organic compounds are present, step ii) further comprises determining the amount of the one or more additional volatile organic compounds and comparing the amount with one or more control values for each of the one or more additional volatile organic compounds; wherein an increase or decrease in the level of said one or more additional volatile organic compounds relative to the control value is indicative of the probability that the individual has colorectal cancer.

In another particular embodiment of the present invention, step i) further comprises analysing the stool sample collected from said individual to detect for the presence of two or more additional volatile organic compounds and, if two or more additional volatile organic compounds are present, step ii) further comprises determining the amount of the two or more additional volatile organic compounds and comparing the amount with two or more control values for each of the two or more additional volatile organic compounds; wherein an increase or decrease in the level of said two or more additional volatile organic compounds relative to the control value is indicative of the probability that the individual has colorectal cancer.

It will be appreciated that the one or more additional volatile organic compounds may be any suitable organic compound that has a high vapour pressure (volatility) at ambient temperature and pressure.

In an embodiment, the one or more additional volatile organic compounds are organic compounds with a molecular weight of less than or equal to 500 Da. Suitably, the one or more additional volatile organic compounds are organic compounds with a molecular weight of less than or equal to 300 Da. More suitably, the one or more additional volatile organic compounds are organic compounds with a molecular weight of less than or equal to 250 Da. Yet more suitably, the one or more additional volatile organic compounds are organic compounds with a molecular weight of less than or equal to 200 Da. Most suitably, the one or more additional volatile organic compounds are organic compounds with a molecular weight of less than or equal to 150 Da.

In another embodiment, the one or more additional volatile organic compounds are organic compounds with a boiling point of less than or equal to 250 °C at a pressure of 1 atmosphere. More suitably, the one or more additional volatile organic compounds are organic compounds with a boiling point of less than or equal to 200 °C at a pressure of 1 atmosphere. More suitably, the one or more additional volatile organic compounds are organic compounds with a boiling point of less than or equal to 175 °C at a pressure of 1 atmosphere. Most suitably, the one or more additional volatile organic compounds are organic compounds with a boiling point of less than or equal to 150 °C at a pressure of 1 atmosphere.

It will be understood that in instances where two or more volatile organic compounds are present in the stool sample of the individual, the two or more volatile organic compounds may form an azeotrope. Thus, it will be understood that the boiling points described in paragraph [0031] above also cover boiling points of azeotropic mixtures of two or more volatile organic compounds.

In another embodiment, the one or more additional volatile organic compounds are selected from:
i) 2-hexanone;
ii) Butanoic acid, 3-methyl-, ethyl ester;
iii) Butanoic acid, 1-methylethyl ester;
iv) Pentanoic acid, 1-methylethyl ester;
v) p-Xylene;
vi) Propanoic acid, 1-methylethyl ester; or
vii) Dimenthol.

Suitably, the one or more additional volatile organic compounds are selected from:
i) 2-hexanone;
ii) Butanoic acid, 3-methyl-, ethyl ester;
iii) Butanoic acid, 1-methylethyl ester;
iv) Pentanoic acid, 1-methylethyl ester;
v) p-Xylene; or
vi) Propanoic acid, 1-methylethyl ester.

More suitably, the one or more additional volatile organic compounds are selected from:
i) 2-hexanone;
ii) Butanoic acid, 3-methyl-, ethyl ester;
iii) Butanoic acid, 1-methylethyl ester; or
iv) Pentanoic acid, 1-methylethyl ester.

Most suitably, the one or more additional volatile organic compounds are selected from:
i) 2-hexanone; or
ii) Butanoic acid, 3-methyl-, ethyl ester.

In another particular embodiment of the present invention, step i) comprises analysing the stool sample collected from said individual to detect for the presence of the volatile organic compound isopropanol and all of the additional volatile organic compounds listed in any one of paragraphs [0027] to [0030] and [0033] to [0036] above and, if all of the volatile organic compounds are present, step ii) further comprises determining the amount of all of the additional volatile organic compounds and comparing the amount with control values for each of the volatile organic compounds; wherein an increase or decrease in the level of the additional volatile organic compounds relative to the control value is indicative of the probability that the individual has colorectal cancer.

In another embodiment, the individual has one or more symptoms associated with colorectal cancer. Symptoms commonly associated with colorectal cancer will be apparent to the person skilled in the art. A non-limiting list of symptoms commonly associated with colorectal cancer includes rectal bleeding, diarrhoea, constipation, abdominal cramps, abdominal pain, fatigue and/or weight loss.

In yet another embodiment, the individual has a predetermined risk factor associated with colorectal cancer.

The term "predetermined risk factor" used herein will be understood as referring to any attribute and/or characteristic an individual possesses prior to the method of the present invention that may increase their likelihood of developing colorectal cancer.

In an embodiment, the predetermined risk factor associated with colorectal cancer is determined based on one or more of the following:
i) a positive Faecal Occult Blood Test (FOBT);
ii) the presence of colorectal polyps;
iii) iron deficiency anaemia;
iv) a condition requiring radiological imaging of the bowel;
v) abnormal bowel habits of the individual (e.g. constipation and/or diarrhoea);
vi) a family history of colorectal polyps; or
vii) the presence of a polyposis syndrome.

In an embodiment, the predetermined risk factor associated with colorectal cancer is determined based on a positive Faecal Occult Blood Test (FOBT).

It will be understood that the method of the present invention suitably encompasses methods of determining the probability that an individual has any form and/or mutation of colorectal cancer. Thus, it will be appreciated that the present invention encompasses methods of determining the probability that an individual has one or more of the following cancer (tumour) types:
- colorectal adenocarcinomas;
- carcinoid tumors;
- colorectal lymphomas;
- gastrointestinal stromal tumors (GISTs);
- leiomyosarcomas; or
- and colorectal melanomas.

In an embodiment, the present invention provides a method of determining the probability that an individual has one or more colorectal adenocarcinomas, carcinoid tumors and/or gastrointestinal stromal tumors (GISTs), as defined hereinabove.

In another embodiment, the present invention provides a method of determining the probability that an individual has one or more colorectal adenocarcinomas, as defined hereinabove.

### Control group

It will be understood that the size and demographic of the control group used to obtain the control values utilised in the method of the present invention may vary. Preferably, the control group is as large and diverse as possible, comprising roughly equal numbers of male and female participants. However, the demographic and ethnicity of the control group may vary according to factors, such as, the location (region) in which method is to be implemented.

In an embodiment, the control group comprises greater than or equal to 30 patients who have displayed one or more colorectal symptoms but, following a colonoscopy, a CT colonography, an MRI colonography and/or a barium enema, were subsequently determined to be cancer free. Suitably, the control group comprises greater than or equal to 40 patients who have displayed one or more colorectal symptoms but, following a colonoscopy, a CT colonography, an MRI colonography and/or a barium enema, were subsequently determined to be cancer free. More suitably, the control group comprises greater than or equal to 50 patients who have displayed one or more colorectal symptoms but, following a colonoscopy, a CT colonography, an MRI colonography and/or a barium enema, were subsequently determined to be cancer free. More suitably, the control group comprises greater than or equal to 60 patients who have displayed one or more colorectal symptoms but, following a colonoscopy, a CT colonography, an MRI colonography and/or a barium enema, were subsequently determined to be cancer free. Most suitably, the control group comprises greater than or equal to 100 patients who have displayed one or more colorectal symptoms but, following a colonoscopy, a CT colonography, an MRI colonography and/or a barium enema, were subsequently determined to be cancer free.

In another embodiment, the control group comprises between 25% and 75% male participants. Suitably, the control group comprises between 35% and 65% male participants. More suitably, the control group comprises between 40% and 60% male participants. Most suitably, the control group comprises between 40% and 55% male participants.

In another embodiment, the control group comprises between 25% and 75% female participants. Suitably, the control group comprises between 35% and 65% female participants. More suitably, the control group comprises between 40% and 60% female participants. Most suitably, the control group comprises between 40% and 55% female participants.

It will be appreciated that the average age of the control group may vary, however given that colorectal cancer is more prevalent in individuals over the age of 50, the mean average age of the control group of the present invention is suitably greater than or equal to 50 (e.g. greater than or equal to 55).

### Particular embodiments

In a particular embodiment, the method of the present invention comprises the steps of:
i) analysing a stool sample collected from said individual to detect whether the volatile organic compounds, isopropanol and butanoic acid, 3-methyl-, ethyl ester, are present; and
ii) if isopropanol and butanoic acid, 3-methyl-, ethyl ester are present, determining the amount of isopropanol and butanoic acid, 3-methyl-, ethyl ester present and comparing these amounts with control values for each respective volatile organic compound;
wherein an increased amount of isopropanol and butanoic acid, 3-methyl-, ethyl ester compared to their respective control values is indicative of an increased probability that the individual has colorectal cancer.

In another particular embodiment, the method of the present invention comprises the steps of:
i) analysing a stool sample collected from said individual to detect whether the volatile organic compounds, isopropanol, butanoic acid, 3-methyl-, ethyl ester, and one or more additional volatile organic compounds are present; and
ii) if isopropanol, butanoic acid, 3-methyl-, ethyl ester one or more additional volatile organic compounds are present, determining the amount of isopropanol, butanoic acid, 3-methyl-, ethyl ester and one or more additional volatile organic compounds present and comparing these amounts with control values for each respective volatile organic compound;

wherein an increased amount of isopropanol and butanoic acid, 3-methyl-, ethyl ester compared to their respective control values is indicative of an increased probability that the individual has colorectal cancer;
and an increase or decrease in the level of said one or more additional volatile organic compounds relative to the control value is indicative of an increased probability that the individual has colorectal cancer.

In another particular embodiment, the method of the present invention comprises the steps of:
i) analysing a stool sample collected from said individual to detect whether the volatile organic compounds, isopropanol, 2-hexanone and butanoic acid, 3-methyl-, ethyl ester, are present; and
ii) if isopropanol, 2-hexanone and butanoic acid, 3-methyl-, ethyl ester are present, determining the amount of isopropanol, 2-hexanone and butanoic acid, 3-methyl-, ethyl ester present and comparing these amounts with control values for each respective volatile organic compound;
wherein an increased amount of isopropanol, 2-hexanone and butanoic acid, 3-methyl-, ethyl ester compared to their respective control values is indicative of an increased probability that the individual has colorectal cancer.

In another particular embodiment, the method of the present invention comprises the steps of:
i) analysing a stool sample collected from said individual to detect whether the volatile organic compounds, isopropanol, 2-hexanone, butanoic acid, 3-methyl-, ethyl ester, and one or more additional volatile organic compounds are present; and
ii) if isopropanol, 2-hexanone, butanoic acid, 3-methyl-, ethyl ester one or more additional volatile organic compounds are present, determining the amount of isopropanol, 2-hexanone, butanoic acid, 3-methyl-, ethyl ester and one or more additional volatile organic compounds present and comparing these amounts with control values for each respective volatile organic compound;

wherein an increased amount of isopropanol, 2-hexanone and butanoic acid, 3-methyl-, ethyl ester compared to their respective control values is indicative of an increased probability that the individual has colorectal cancer;
and an increase or decrease in the level of said one or more additional volatile organic compounds relative to the control value is indicative of an increased probability that the individual has colorectal cancer.

In another particular embodiment, the method of the present invention comprises the steps of:
i) analysing a stool sample collected from said individual to detect whether the volatile organic compounds, isopropanol, 2-hexanone, butanoic acid, 3-methyl-, ethyl ester, and one or more additional volatile organic compounds are present; and
ii) if isopropanol, 2-hexanone, butanoic acid, 3-methyl-, ethyl ester and one or more additional volatile organic compounds are present, determining the amount of isopropanol, 2-hexanone, butanoic acid, 3-methyl-, ethyl ester and one or more additional volatile organic compounds present and comparing these amounts with control values for each respective volatile organic compound;
wherein the one or more additional volatile organic compounds are selected from:
i) Butanoic acid, 1-methylethyl ester;
ii) Pentanoic acid, 1-methylethyl ester;
iii) p-Xylene;
iv) Propanoic acid, 1-methylethyl ester; or
v) Dimenthol;

and wherein an increased amount of isopropanol, 2-hexanone and butanoic acid, 3-methyl-, ethyl ester compared to their respective control values is indicative of an increased probability that the individual has colorectal cancer;
and an increase or decrease in the level of said one or more additional volatile organic compounds relative to the control value is indicative of an increased probability that the individual has colorectal cancer.

In another particular embodiment, the method of the present invention comprises the steps of:
i) analysing a stool sample collected from said individual to detect whether each of the volatile organic compounds listed below are present:
   ▪ isopropanol;
   ▪ 2-hexanone;
   ▪ butanoic acid, 3-methyl-, ethyl ester;
   ▪ butanoic acid, 1-methylethyl ester;
   ▪ pentanoic acid, 1-methylethyl ester;
   ▪ p-xylene;
   ▪ propanoic acid, 1-methylethyl ester; and
   ▪ dimenthol;
ii) if each of the volatile organic compounds of step i) are present, determining the amount of each of the volatile organic compounds of step i) and comparing these amounts with control values for each respective volatile organic compound;

and wherein an increased amount of isopropanol, 2-hexanone, butanoic acid, 3-methyl-, ethyl ester, butanoic acid, 1-methylethyl ester, pentanoic acid, 1-methylethyl ester, p-xylene and propanoic acid, 1-methylethyl ester compared to their respective control values is indicative of an increased probability that the individual has colorectal cancer;
and a decrease in the level of dimenthol relative to the control value is indicative of an increased probability that the individual has colorectal cancer.

In another particular embodiment, the method of the present invention comprises the steps of:
i) analysing a stool sample collected from said individual to detect whether each of the volatile organic compounds listed below are present:
   ▪ isopropanol;
   ▪ 2-hexanone;
   ▪ butanoic acid, 3-methyl-, ethyl ester;
   ▪ butanoic acid, 1-methylethyl ester;
   ▪ pentanoic acid, 1-methylethyl ester; or
   ▪ p-xylene;
i) if each of the volatile organic compounds of step i) are present, determining the amount of each of the volatile organic compounds of step i) and comparing these amounts with control values for each respective volatile organic compound;
and wherein an increased amount of isopropanol, 2-hexanone, butanoic acid, 3-methyl-, ethyl ester, butanoic acid, 1-methylethyl ester, pentanoic acid, 1-methylethyl ester and p-xylene compared to their respective control values is indicative of an increased probability that the individual has colorectal cancer.

### Biomarkers of the present invention

Following extensive investigation, the inventors were able to identify and quantify the amounts of specific volatile organic compounds (e.g. isopropanol) present in a stool sample obtained from an individual. Through the correlation of the amounts of each of these volatile organic compounds with corresponding amounts of each volatile organic compound in the stools of a control group, the inventors were able to determine the probability of an individual having colorectal cancer. Thus, the inventors identified certain volatile organic compounds described herein, used alone or in combination, could be used as biomarkers for colorectal cancer.

Accordingly, in a further aspect of the present invention, there is provided a use of isopropanol in a stool sample as a biomarker for colorectal cancer.

In yet another aspect of the present invention, there is provided a use of isopropanol and one or more additional volatile organic compounds in a stool sample as biomarkers for colorectal cancer, wherein the one or more additional volatile organic compounds are selected from:
i) 2-hexanone;
ii) Butanoic acid, 3-methyl-, ethyl ester;
iii) Butanoic acid, 1-methylethyl ester;
iv) Pentanoic acid, 1-methylethyl ester;
v) p-Xylene;
vi) Propanoic acid, 1-methylethyl ester; or
vii) dimenthol.

In certain embodiments, isopropanol, butanoic acid, 3-methyl-, ethyl ester and optionally one or more other volatile organic compound listed in paragraph [0054] or [0060] above are used as biomarkers for colorectal cancer.

In other embodiments, isopropanol, 2-hexanone, butanoic acid, 3-methyl-, ethyl ester and optionally one or more other volatile organic compound listed in paragraph [0054] or [0060] above are used as biomarkers for colorectal cancer.

In a particular embodiment, isopropanol and all of the other volatile organic compounds listed in paragraph [0054] or [0060] above are used as biomarkers for colorectal cancer.

The ability of the volatile organic compound (e.g. isopropanol) of the present invention to function as a reliable biomarker for colorectal cancer may be quantified using various statistic models and/or calculations. The person skilled in the art will be able to select one or more suitable statistical models or calculations to use in order to determine the effectiveness of the volatile organic compound(s) as a predictor of colorectal cancer. One particular statistical model which may be used to quantify the ability of the volatile organic compound(s) (e.g. isopropanol) to function as a biomarker for colorectal cancer is the Receiver Operating Characteristic Curve, which is created by plotting the true positive rate (TPR) against the false positive rate (FPR) at various threshold settings. Calculating the area under the Receiver Operating Characteristic (commonly referred to as the AUROC) gives a quantitative value for how good a predictor the particular volatile organic compound is.

Thus, in an embodiment, the volatile organic compound(s) of the present invention, used alone or in combination, have a calculated Area under Receiver Operating Characteristic (AUROC) value of greater than or equal to 0.7. Suitably, the volatile organic compound(s) of the present invention, used alone or in combination, have a calculated Area under Receiver Operating Characteristic (AUROC) value of greater than or equal to 0.75. More suitably, the volatile organic compound(s) of the present invention, used alone or in combination, have a calculated Area under Receiver Operating Characteristic (AUROC) value of greater than or equal to 0.8. Most suitably, the volatile organic compound(s) of the present invention, used alone or in combination, have a calculated Area under Receiver Operating Characteristic (AUROC) value of greater than or equal to 0.85.

Another statistical model which may be used to quantify the ability of the volatile organic compound (e.g. isopropanol) to function as a biomarker for colorectal cancer is the Student's t-test. This test provides a method of testing hypotheses about the mean average of a small sample drawn from a larger distributed population, when the standard deviation of the larger population is unknown. The Student's t-test provides certain P values, or calculated probabilities, which are the probabilities, assuming the null hypothesis is true, of observing a more extreme test statistic in the direction of the alternative hypothesis than the one observed. A result is said to be statistically significant if the p value is small (i.e. less than 0.1).

Thus, in another embodiment, the volatile organic compound(s) of the present invention have a p value as calculated using the Student's t-test of less than or equal to 0.06. Suitably, the volatile organic compound(s) of the present invention have a p value as calculated using the Student's t-test of less than or equal to 0.05. More suitably, the volatile organic compound(s) of the present invention have a p value as calculated using the Student's t-test of less than or equal to 0.04. Yet more suitably, the volatile organic compound(s) of the present invention have a p value as calculated using the Student's t-test of less than or equal to 0.03. Most suitably, the volatile organic compound(s) of the present invention have a p value as calculated using the Student's t-test of less than or equal to 0.01.

### EXAMPLES

### Description of drawings

Embodiments of the invention will be described, by way of example only, with reference to the accompanying drawings, in which:
Figure 1 shows the PLS-DA comparing VOCs emitted from faecal samples from patients with colonic adenocarcinoma and no neoplasia (normal). All identified VOCs were subjected to PLS-DA using Metaboanalyst.
Figure 2 shows an ROC curve showing the sensitivity and specifcity of the 3-VOC model (isopropyl alcohol; 2-hexanone; butanoic acid 3-methyl, ethyl ester) for the diagnosis of colorectal cancer.

### Materials and methods

### Participants

Most participants were recruited from colonoscopy waiting lists at the Royal Liverpool University Hospital.

Participants were people referred by the Merseyside and Wirral Bowel Cancer Screening Programme with positive FOBt, or patients undergoing colonoscopy for polyp surveillance, planned polypectomy, the investigation of iron deficiency anaemia (IDA), change in bowel habit or abnormal radiological imaging. The FOBt status of the non-BCSP patients was unknown.

A subset of the faecal samples were provided from a cohort of symptomatic patients undergoing colonoscopy in Sheffield and Plymouth, UK.

Ethics committee approval for the study was obtained from the NRES Committee South West - Central Bristol (REC reference 14/SW/1162) with R&D approval from University of Liverpool and Broadgreen University Hospital Trust from where patients were recruited over a 12-month period. All patients were supplied with an information sheet and provided written consent.

Specific permission was also granted by the NHS Bowel Cancer Screening Programme Research Committee Samples collected from Sheffield and Plymouth were acquired in line with existing ethical approval.

### Sample collection and storage

Samples were produced within 48 hours of the donor attending their colonoscopy and before commencing the required bowel preparation. Participants were asked to place at least 3 spoonfuls of faeces into a glass vial, before it was sealed and stored in a cool place. The sample was brought to the Endoscopy Department when the patients attended for the colonoscopy, and collected by the researcher. Colonoscopy results, including any histological findings, were recorded. Patients were then categorised as having no neoplasia, adenomatous polyp(s) or cancer. The location, size and number of polyps was recorded. Polyps were assigned to the adenoma group only after histological confirmation. Hyperplastic polyps were classified as no neoplasia.

Demographic details, smoking status and antibiotic use was also recorded

### Laboratory analysis

Samples were taken from the Endoscopy Department directly to the laboratory and 450mg of unadulterated faeces aliquoted into a new 10ml headspace vials and sealed with magnetic caps (Supelco, UK).^{[19]} Both the sample intended for analysis, and the residual faeces, were then stored at -20°C until GCMS analysis was performed.

Headspace VOCs analysis was performed using a Combipal (CTC, Switzerland) and carboxen/ polydimethylsiloxane solid phase microextraction fibre (Sigma Aldrich, Dorset, UK). The fibre was exposed to the headspace above the faeces for 20 minutes.

VOCs were analysed by GCMS (Perkin Elmer Clarus 500 quadrupole, Beaconsfield, UK): VOCs were thermally desorbed from the fibre at 220°C in the injection port of the GCMS for 5 minutes. Injection was made in splitless mode and a split of 50 ml/min was turned on two minutes into the run. Helium carrier gas of 99.996% purity (BOC, Guildford, UK) was passed through a helium purification system, Excelasorb^{™} (Supelco, Poole, UK) at 1 ml min⁻¹. The GC column was a 60 metre long Zebron ZB-624 capillary column with an inner diameter of 0.25 mm. The column was lined with a 1.4 µm think film, specifically designed for the separation of VOCs (Phenomenex, Macclesfield, UK) consisting of 94% dimethyl polysiloxane and 6% cyanopropylphenyl. The GCMS temperature program of the run was as follows: initial oven temperature was held at 40°C for 2 minutes then the temperature was ramped up at a rate of 5°C/min to 220°C, with a 4 minute hold at this temperature to give a total run time of 42 minutes. The mass spectrometer was run in electron impact (EI) ionization mode, scanning the mass ion range 10-300 at 0.05 scan /sec. A 4 minute solvent delay was used at the start of the run. ^{[19-21]}

### Data processing

The GCMS data were processed using a pipeline involving the Automated Mass Spectral Deconvolution and Identification System software (AMDIS, Version 2.71, 2012), the NIST mass spectral library (version 2.0, 2011) and the R (R core team, 2013) package Metab.^{[2]}

AMDIS and NIST software were used to build a VOC library containing 162 metabolites present in the stool samples analysed in this study. A forward and reverse match of 800/1000 and above was used for assigning tentative compound identifications. Using this VOC library, AMDIS was then applied to deconvolute chromatograms and identifying metabolites.

The report generated by AMDIS was further processed by Metab in order to confirm the identity of metabolites and recalculate their relative abundances based on the intensity of a specific ion mass fragment per metabolite. In order to develop robust parsimonious statistical models, those compounds found to be present in fewer than 20% of the patients in both groups were removed.^{[20,21]}

### Statistical analysis

Data analysis was performed in R, Stata and Metaboanalyst,^{[3]} utilising Student's t test, Fisher's exact test, ANOVA, false discovery rate correction, Partial Least Squared Discriminant Analysis (PLS-DA), factor analysis and Receiver Operator Characteristic (ROC) analysis.

Logistic regression modelling, along with 10-fold cross-validation was used to test potential biomarkers.

When Metaboanalyst was used the data was normalised by median and log transformed.

### Results

### Participant demographic

There were a total of 137 patients included in this study: the average age was 64.3 years; 56% were male. The mean age was least in those with no neoplasia and greatest those the cancer, *p*=*0.02.*

None of the participants reported being smokers or vegetarians.

Self-reported ethnicity was noted: all but one patient reported themselves to be White British. BCSP participants were the largest group of donors (31.5%).

**Table 1 - Demographic and clinical features of participants recruited in Liverpool, Sheffield and Plymouth.**

| | **Total** | **No-neoplasia** | **Adenoma** | **Cancer** |
|---|---|---|---|---|
| **Number** | 137 | 60 | 56 | 21 |
| **Mean age, years (range)** | 64.3 | 61.9 | 65.6 | 72.7 |
| | (22-85) | (22-85) | (41-84) | (64-78) |

| **Gender** | | | | |
|---|---|---|---|---|
| Male | 69 | 25 | 36 | 7 |
| Female | 57 | 34 | 20 | 3 |
| **Smoker (Yes)** | 0 | 0 | 0 | 0 |

| **Indication for colonoscopy** | | | | |
|---|---|---|---|---|
| BCSP | 38 | 13 | 22 | 3 |
| IDA | 27 | 16 | 6 | 5 |
| Change in bowel habit-diarrhoea | 16 | 11 | 4 | 1 |
| Surveillance previous neoplasia/FH | 35 | 10 | 24 | 1 |
| IBD assessment/surveillance | 9 | 9 | 0 | 0 |
| G! bleeding | 1 | 1 | 0 | 0 |
| Unknown | 11 | 0 | 0 | 11 |

### Volatile organic compound (biomarker) identification

A total of 162 VOCs were identified in the whole sample set. The mean number of VOCs identified in the entire cohort was 56.7, with no significant difference in those with or without neoplasia, *p*=*0.2.*

Biomarker identification focused on higher risk neoplastic disease, namely established colorectal cancer, a single adenoma >1cm in size and >4 individual polyps of any size.

Analysis was performed using quantitative and qualitative data.

### VOCs as a biomarker for colonic adenocarcinoma- Quantitative analysis

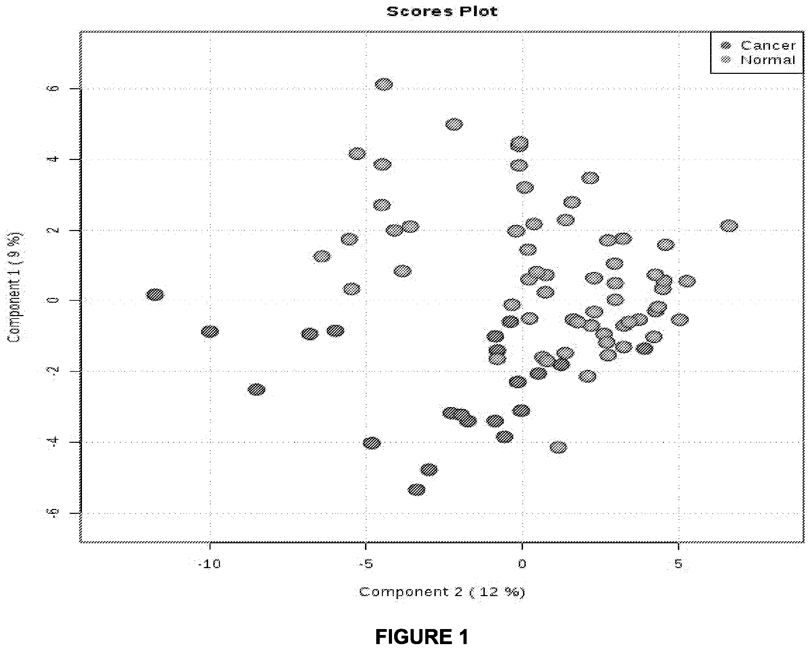

PLS-DA comparing those with no neoplasia and those with colonic adenocarcinoma showed a separation that suggested potential diagnostic utility (Figure 1). Potential candidates for biomarker analysis can be seen in Table 2.

**Table 2 - VOCs with p values <0.05, identified from Student's t test comparing no neoplasia and cancer. The q value was found after correcting for multiple comparisons.**

| **VOC** | **p value** | **q value** | **Abundance direction in cancer:** |
|---|---|---|---|
| Isopropanol | <0.0001 | 0.004 | Increased |
| 2-hexanone | 0.01 | 0.77 | Increased |
| Butanoic acid, 3-methyl-, ethyl ester | 0.03 | 0.77 | Increased |
| Butanoic acid, 1-methylethyl ester | 0.03 | 0.77 | Increased |
| Pentanoic acid, 1-methylethyl ester | 0.03 | 0.77 | Increased |
| p-Xylene | 0.03 | 0.77 | Increased |
| Propanoic acid, 1-methylethyl ester | 0.04 | 0.77 | Increased |
| dl-Menthol | 0.05 | 0.77 | Decreased |

Isopropanol showed the most promise as a biomarker for colorectal cancer. In isolation, it achieves an Area under Receiver Operating Characteristic (AUROC) of 0.76.

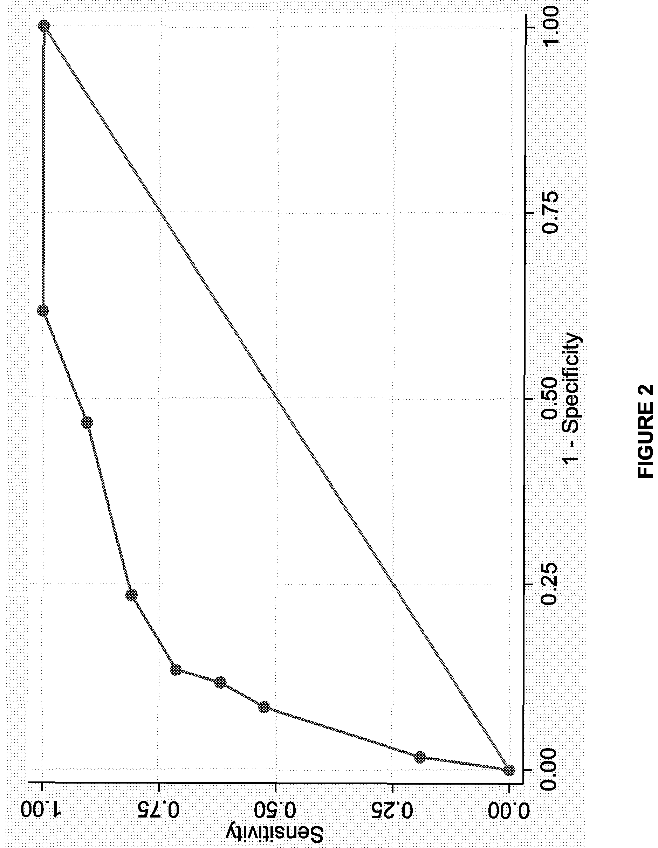

Calculating ratios of all possible metabolite pairs and then choosing top ranked ratios, based on p values, allowed for further biomarker assessment. This technique identified the combination of butanoic acid, 3-methyl- and isopropanol as having the greatest potential: data from 21 patients with cancer and 60 with no neoplasia were modelled using logistic regression and 10-fold cross-validation, based upon the abundance of butanoic acid, 3-methyl- and isopropanol (Figure 2): AUROC is 0.86, sensitivity 87.9% (95% Cl 0.87-0.99) and specificity 84.6% (95% Cl 0.65-1.0).

### VOCs as a biomarker for colonic adenocarcinoma- Qualitative analysis

Principle components analysis and a non-orthogonal rotation feature analysis was applied to qualitative (presence/absence) data for VOCs. The solution could not be extracted due to convergence issues until the number of extracted factors had been reduced to 17. This process highlighted the combination of isopropanol, 2-hexanone and 3-methyl- butanoic acid ethyl ester as a key predictor. Using all 3 VOCs as a biomarker panel predicts cancer versus all others with a p value of 0.001 and an area under the curve of 0.73, and predicts cancer versus normal with a p value of 0.006 and an area under the ROC curve of 0.70.

**Table 3 - Prevalence of isopropanol, 2-hexanone and 3-methyl- butanoic acid ethyl ester in stool from patients with colonic adenocarcinoma, adenomatous colonic polyps and no neoplasia.**

| | **Adenoma** | **Cancer** | **Normal** |
|---|---|---|---|
| Mean number of these three VOC's | 1.16 | 2.0 | 1.33 |
| Proportion with none of these three VOCs | 23.2% (13/56) | 0% (0/21) | 20.0% (12/60) |
| With just 1 | 42.8% (24/56) | 23.8% (5/21) | 36/7% (22/60) |
| With just 2 | 28.6% (16/56) | 52.4% (11/21) | 33.3% (20/60) |
| With all three | 5.4% (3/56) | 23.8% (5/21) | 10.0% (6/60) |

Pure reference solutions of isopropanol, 2-hexanone and butanoic acid, 3-methyl-, ethyl ester confirmed the identification within the stool samples was correct.

While specific embodiments of the invention have been described herein for the purpose of reference and illustration, various modifications will be apparent to a person skilled in the art without departing from the scope of the invention as defined by the appended claims.

### REFERENCES

1 CRUK. http://publications.cancerresearchuk.org/downloads/Product/CS_KF_BOWEL.pdf. 2014.
2 Aggio R, Villas-Bôas SG, Ruggiero K. Metab: an R package for high-throughput analysis of metabolomics data generated by GC-MS. Bioinformatics 2011;27:2316-8. doi: 10.1093/bioinformatics/btr379
3 Xia, J., Sinelnikov, I., Han, B., and Wishart D. MetaboAnalyst 3.0 - making metabolomics more meaningful . Nucleic acid Res 2015;43:W251-7.
4 Ferlay J, Steliarova-Foucher E, Lortet-Tieulent J, et al. Cancer incidence and mortality patterns in Europe: estimates for 40 countries in 2012. Eur J Cancer 2013;49:1374-403. doi:10.1016/j.ejca.2012.12.027
5 Rees CJ, Bevan R. The National Health Service Bowel Cancer Screening Program: the early years. Expert Rev Gastroenterol Hepatol 2013;7:421-37. doi:10.1586/17474124.2013.811045
6 Trueman P, Lowson K, Chaplin S, et al. Bowel Cancer Services: Costs and Benefits. 2007.
7 Logan RF a, PatnickJ, Nickerson C, etal. Outcomes of the Bowel Cancer Screening Programme (BCSP) in England after the first 1 million tests. Gut 2012;61:1439-46. doi:10.1136/gutjnl-2011-300843
8 Corley DA, Jensen CD, Marks AR, et al. Adenoma detection rate and risk of colorectal cancer and death. N Engl J Med 2014;370:2541. doi:10.1056/NEJMoa1309086
9 Altobelli E, Lattanzi a, Paduano R, et al. Colorectal cancer prevention in Europe: burden of disease and status of screening programs. Prev Med (Baltim) 2014;62:132-41. doi:10.1016/j.ypmed.2014.02.010
10 Colorectal cancer association of Canada. A guide to FOBT and FIT tests. Guaiac-based FOBT Immunochem. FOBT. 2016;:http://www.colorectal-cancer.ca/en/screening/fobt-.
11 Tonus C, Neupert G, Sellinger M. Colorectal cancer screening by non-invasive metabolic biomarker fecal tumor M2-PK. World J Gastroenterol 2006;12:7007-11.
12 Tinmouth J, Lansdorp-Vogelaar I, Allison JE. Faecal immunochemical tests versus guaiac faecal occult blood tests: what clinicians and colorectal cancer screening programme organisers need to know. Gut 2015;64:1327-37. doi:10.1136/gutjnl-2014-308074
13 Zorzi M, Fedeli U, Schievano E, et al. Impact on colorectal cancer mortality of screening programmes based on the faecal immunochemical test. Gut 2015;64:784-90. doi: 10.1136/g utjn I-2014-307508
14 Sawbridge D, Probert C. Population-based screening in colorectal cancer - current practice and future developments: faecal biomarkers review. J Gastrointestin Liver Dis 2014;23:195-202.
15 Batty CA, Cauchi M, Lourenço C, et al. Use of the Analysis of the Volatile Faecal Metabolome in Screening for Colorectal Cancer. PLoS One 2015;10:e0130301. doi:10.1371/journal.pone.0130301
16 Arasaradnam RP, McFarlane MJ, Ryan-Fisher C, et al. Detection of colorectal cancer (CRC) by urinary volatile organic compound analysis. PLoS One 2014;9:e108750. doi:10.1371/journal.pone.0108750
17 Altomare DF, Di Lena M, Porcelli F, et al. Exhaled volatile organic compounds identify patients with colorectal cancer. Br J Surg 2013;100:144-50. doi:10.1002/bjs.8942
18 Altomare DF, Di Lena M, Porcelli F, et al. Effects of Curative Colorectal Cancer Surgery on Exhaled Volatile Organic Compounds and Potential Implications in Clinical Follow-up. Ann Surg 2015;262:862-7. doi:10.1097/SLA.0000000000001471
19 Reade S MA. Optimisation of Sample Preparation for Direct SPME-GC-MS Analysis of Murine and Human Faecal Volatile Organic Compounds for Metabolomic Studies. J Anal Bioanal Tech 2014;5. doi:10.4172/2155-9872.1000184
20 Bond A, Vernon A, Reade S, et al. Investigation of Volatile Organic Compounds Emitted from Faeces for the Diagnosis of Giardiasis. J Gastrointest Liver Dis 2015;24:281-6.
21 Khalid T, Aggio R, White P, et al. Urinary Volatile Organic Compounds for the Detection of Prostate Cancer. PLoS One 2015;10:e0143283. doi:10.1371/journal.pone.0143283

## Claims

1. A method of determining the probability that an individual has colorectal cancer, the method comprising the steps of:
i) analysing a stool sample collected from said individual to detect whether the volatile organic compound isopropanol is present;
ii) if isopropanol is present in step i), determining the amount of isopropanol that is present and comparing the amount detected with a control value;
wherein the presence of an increased amount of isopropanol in the stool compared to the control value is indicative of an increased probability that the individual has colorectal cancer.

2. A method according to claim 1, wherein step i) further comprises analysing the stool sample collected from said individual to detect for the presence of one or more additional volatile organic compounds and, if one or more additional volatile organic compounds are present, step ii) further comprises determining the amount of the one or more additional volatile organic compounds and comparing the amount detected with one or more control values for each of the one or more additional volatile organic compounds;
wherein an increase or decrease in the level of said one or more additional volatile organic compounds relative to the control value is indicative of the probability that the individual has colorectal cancer.

3. A method according to claim 2, wherein the one or more additional volatile organic compounds are organic compounds with:
(i) a molecular weight of less than or equal to 250 Da; and/or
(ii) a boiling point of less than or equal to 250 °C when measured at a standard atmospheric pressure of 101.3 kPa (1 atmosphere).

4. A method according to any one of claims 2 or 3, wherein the one or more additional volatile organic compounds are selected from:
i) 2-hexanone;
ii) Butanoic acid, 3-methyl-, ethyl ester;
iii) Butanoic acid, 1-methylethyl ester;
iv) Pentanoic acid, 1-methylethyl ester;
v) p-Xylene;
vi) Propanoic acid, 1-methylethyl ester; or
vii) Dimenthol.

5. A method according to any preceding claim, wherein said method comprises the steps of:
i) analysing a stool sample collected from said individual to detect whether the volatile organic compounds, isopropanol and butanoic acid, 3-methyl-, ethyl ester, are present; and
ii) if isopropanol and butanoic acid, 3-methyl-, ethyl ester are present, determining the amount of isopropanol and butanoic acid, 3-methyl-, ethyl ester present and comparing these amounts with control values for each respective volatile organic compound;
wherein an increased amount of isopropanol and butanoic acid, 3-methyl-, ethyl ester compared to their respective control values is indicative of an increased probability that the individual has colorectal cancer;
optionally wherein step i) further comprises analysing the stool sample collected from said individual to detect the presence of one or more additional volatile organic compounds and, if one or more additional volatile organic compounds are present, step ii) further comprises determining the amount of the one or more additional volatile organic compounds and comparing the amount with one or more control values for each of the one or more additional volatile organic compounds;
wherein an increase or decrease in the level of said one or more additional volatile organic compounds relative to the control value is indicative of the probability that the individual has colorectal cancer;
further optionally wherein the one or more additional volatile organic compounds are selected from
i) 2-hexanone;
ii) Butanoic acid, 1-methylethyl ester;
iii) Pentanoic acid, 1-methylethyl ester;
iv) p-Xylene;
v) Propanoic acid, 1-methylethyl ester; or
vi) Dimenthol;
and wherein an increase in the levels of i) to v) relative to their respective control values and a decrease in the amount of vi) relative to its control value is indicative of an increased probability that the individual has colorectal cancer.

6. A method according to any preceding claim, wherein said method comprises the steps of:
i) analysing the stool sample collected from said individual to detect whether the volatile organic compounds, isopropanol, 2-hexanone and butanoic acid, 3-methyl-, ethyl ester, are present;
ii) if isopropanol, 2-hexanone and butanoic acid, 3-methyl-, ethyl ester are present in step i), determining the amount of isopropanol, 2-hexanone and butanoic acid, 3-methyl-, ethyl ester present and comparing the amount with a control values for each respective volatile organic compound;
wherein an increased amount of isopropanol, 2-hexanone and butanoic acid, 3-methyl-, ethyl ester compared to their respective control values is indicative of an increased probability that the individual has colorectal cancer;
optionally wherein step i) further comprises analysing the stool sample collected from said individual to detect the presence of one or more additional volatile organic compounds and, if one or more additional volatile organic compounds are present, step ii) further comprises determining the amount of the one or more additional volatile organic compounds and comparing the amount with one or more control values for each of the one or more additional volatile organic compounds
wherein an increase or decrease in the level of said one or more additional volatile organic compounds relative to the control value is indicative of the probability that the individual has colorectal cancer;
further optionally wherein the one or more additional volatile organic compounds are selected from:
i) Butanoic acid, 1-methylethyl ester;
ii) Pentanoic acid, 1-methylethyl ester;
iii) p-Xylene;
iv) Propanoic acid, 1-methylethyl ester; or
v) Dimenthol;
and wherein an increase in the levels of i) to iv) relative to their respective control values and a decrease in the amount of v) relative to its control value is indicative of an increased probability that the individual has colorectal cancer.

7. A method according to claim 4, wherein the presence and amount of isopropanol and all of the volatile organic compounds listed in claim 4 are determined, and the amounts of each are compared with respective control values.

8. A method according to any preceding claim, wherein the presence and amount of the volatile organic compound is determined using gas chromatography mass spectrometry (GC-MS).

9. A method according to any preceding claim, wherein the individual has one or more symptoms associated with colorectal cancer;
optionally wherein the symptoms associated with colorectal cancer are selected from rectal bleeding, diarrhoea, constipation, abdominal cramps, abdominal pain, fatigue or weight loss.

10. A method according to any preceding claim, wherein the individual has a predetermined risk factor associated with colorectal cancer,
optionally wherein the predetermined risk factor associated with colorectal cancer is determined based on one or more of the following:
i) a positive Faecal Occult Blood Test (FOBT);
ii) the presence of colorectal polyps;
iii) iron deficiency anaemia;
iv) a condition requiring radiological imaging of the bowel;
v) abnormal bowel habits of the individual (e.g. constipation and/or diarrhoea);
vi) a family history of colorectal polyps; or
vii) the presence of a polyposis syndrome.

11. A method according to any one of claims 1 to 10, wherein said method involves determining the probability that an individual has colorectal adenocarcinoma.

12. Use of isopropanol in a stool sample as a biomarker for colorectal cancer.

13. Use according to claim 12, wherein isopropanol and one or more additional volatile organic compounds in a stool sample as biomarkers for colorectal cancer, wherein the one or more additional volatile organic compounds are selected from:
i) 2-hexanone;
ii) Butanoic acid, 3-methyl-, ethyl ester;
iii) Butanoic acid, 1-methylethyl ester;
iv) Pentanoic acid, 1-methylethyl ester;
v) p-Xylene;
vi) Propanoic acid, 1-methylethyl ester; or
vii) dimenthol.

14. Use according to claim 13, wherein isopropanol, butanoic acid, 3-methyl-, ethyl ester and optionally one or more other volatile organic compound listed in claim 13 are used as biomarkers for colorectal cancer.

15. Use according to any one of claims 13 or 14, wherein, isopropanol, 2-hexanone, butanoic acid, 3-methyl-, ethyl ester and optionally one or more other volatile organic compound listed in claim 13 are used as biomarkers for colorectal cancer.

## Patentansprüche

1. Verfahren zum Bestimmen der Wahrscheinlichkeit, dass ein Individuum Dickdarmkrebs hat, das Verfahren umfassend die folgenden Schritte:
i) Analysieren einer von dem Individuum entnommenen Stuhlprobe, um nachzuweisen, ob die flüchtige organische Verbindung Isopropanol vorhanden ist,
ii) wenn in Schritt i) Isopropanol vorhanden ist, Bestimmen der vorhandenen Menge an Isopropanol und Vergleichen der nachgewiesenen Menge mit einem Kontrollwert;
wobei das Vorhandensein einer erhöhten Menge an Isopropanol im Stuhl im Vergleich zu dem Kontrollwert indikativ für eine erhöhte Wahrscheinlichkeit ist, dass das Individuum Dickdarmkrebs hat.

2. Verfahren nach Anspruch 1, wobei Schritt i) ferner ein Analysieren der von dem Individuum entnommenen Stuhlprobe umfasst, um das Vorhandensein einer oder mehrerer zusätzlicher flüchtiger organischer Verbindungen nachzuweisen, und, wenn eine oder mehrere zusätzliche flüchtige organische Verbindungen vorhanden sind, Schritt ii) ferner ein Bestimmen der Menge der einen oder mehreren zusätzlichen flüchtigen organischen Verbindungen und das Vergleichen der nachgewiesenen Menge mit einem oder mehreren Kontrollwerten für jede der einen oder mehreren zusätzlichen flüchtigen organischen Verbindungen umfasst,
wobei eine Zunahme oder Abnahme des Spiegels der einen oder mehreren zusätzlichen flüchtigen organischen Verbindungen in Bezug auf den Kontrollwert indikativ für die Wahrscheinlichkeit ist, dass das Individuum Dickdarmkrebs hat.

3. Verfahren nach Anspruch 2, wobei die eine oder mehreren zusätzlichen flüchtigen organischen Verbindungen organische Verbindungen sind, mit:
(i) einem Molekulargewicht von weniger als oder gleich wie 250 Da; und/oder
(ii) einem Siedepunkt von weniger als oder gleich wie 250 °C, gemessen bei einem atmosphärischen Standarddruck von 101,3 kPa (1 Atmosphäre).

4. Verfahren nach einem der Ansprüche 2 oder 3, wobei die eine oder mehreren zusätzlichen flüchtigen organischen Verbindungen ausgewählt sind aus:
i) 2-Hexanon;
ii) Butansäure, 3-Methyl-, Ethylester,
iii) Butansäure, 1-Methylethylester;
iv) Pentansäure, 1-Methylethylester;
v) p-Xylol;
vi) Propansäure, 1-Methylethylester; oder
vii) Dimenthol.

5. Verfahren nach einem der vorherigen Ansprüche, wobei das Verfahren die folgenden Schritte umfasst:
i) Analysieren einer von dem Individuum entnommenen Stuhlprobe, um nachzuweisen, ob die flüchtigen organischen Verbindungen Isopropanol und Butansäure, 3-Methyl-, Ethylester, vorhanden sind, und
ii) bei Vorhandensein von Isopropanol und Butansäure, 3-Methyl-, Ethylester, Bestimmen der vorhandenen Menge an Isopropanol und Butansäure, 3-Methyl-, Ethylester und Vergleichen dieser Mengen mit den Kontrollwerten für jede jeweilige flüchtige organische Verbindung;
wobei eine erhöhte Menge an Isopropanol und Butansäure, 3-Methyl-, Ethylester im Vergleich zu ihren jeweiligen Kontrollwerten indikativ für eine erhöhte Wahrscheinlichkeit ist, dass das Individuum Dickdarmkrebs hat;
optional, wobei Schritt i) ferner ein Analysieren der von dem Individuum entnommenen Stuhlprobe umfasst, um das Vorhandensein einer oder mehrerer zusätzlicher flüchtiger organischer Verbindungen nachzuweisen, und, wenn eine oder mehrere zusätzliche flüchtige organische Verbindungen vorhanden sind, Schritt ii) ferner ein Bestimmen der Menge der einen oder mehreren zusätzlichen flüchtigen organischen Verbindungen und das Vergleichen der Menge mit einem oder mehreren Kontrollwerten für jede der einen oder mehreren zusätzlichen flüchtigen organischen Verbindungen umfasst,
wobei eine Zunahme oder Abnahme des Spiegels der einen oder mehreren zusätzlichen flüchtigen organischen Verbindungen in Bezug auf den Kontrollwert indikativ für die Wahrscheinlichkeit ist, dass das Individuum Dickdarmkrebs hat;
ferner optional wobei die eine oder mehreren zusätzlichen flüchtigen organischen Verbindungen ausgewählt sind aus
i) 2-Hexanon;
ii) Butansäure, 1-Methylethylester;
iii) Pentansäure, 1-Methylethylester;
iv) p-Xylol;
v) Propansäure, 1-Methylethylester; oder
vi) Dimenthol;
und wobei eine Zunahme der Werte von i) bis v) in Bezug auf ihre jeweiligen Kontrollwerte und eine Abnahme der Menge von vi) in Bezug auf ihren Kontrollwert indikativ für eine erhöhte Wahrscheinlichkeit ist, dass das Individuum Darmkrebs hat.

6. Verfahren nach einem der vorherigen Ansprüche, wobei das Verfahren die folgenden Schritte umfasst:
i) Analysieren der von dem Individuum entnommenen Stuhlprobe, um nachzuweisen, ob die flüchtigen organischen Verbindungen Isopropanol, 2-Hexanon und Butansäure, 3-Methyl-, Ethylester, vorhanden sind,
ii) wenn in Schritt i) Isopropanol, 2-Hexanon und 3-Methyl-Butansäureethylester vorhanden sind, Bestimmen der Menge an vorhandenem Isopropanol, 2-Hexanon und 3-Methyl-Butansäureethylester und Vergleichen der Menge mit einem Kontrollwert für jede entsprechende flüchtige organische Verbindung;
wobei eine erhöhte Menge an Isopropanol, 2-Hexanon und Butansäure, 3-Methyl-, Ethylester im Vergleich zu ihren jeweiligen Kontrollwerten indikativ für eine erhöhte Wahrscheinlichkeit ist, dass das Individuum Dickdarmkrebs hat;
optional, wobei Schritt i) ferner ein Analysieren der von dem Individuum entnommenen Stuhlprobe umfasst, um das Vorhandensein einer oder mehrerer zusätzlicher flüchtiger organischer Verbindungen nachzuweisen, und, wenn eine oder mehrere zusätzliche flüchtige organische Verbindungen vorhanden sind, Schritt ii) ferner ein Bestimmen der Menge der einen oder mehreren zusätzlichen flüchtigen organischen Verbindungen und das Vergleichen der Menge mit einem oder mehreren Kontrollwerten für jede der einen oder mehreren zusätzlichen flüchtigen organischen Verbindungen umfasst
wobei eine Zunahme oder Abnahme des Spiegels der einen oder mehreren zusätzlichen flüchtigen organischen Verbindungen in Bezug auf den Kontrollwert indikativ für die Wahrscheinlichkeit ist, dass das Individuum Dickdarmkrebs hat;
ferner optional, wobei die eine oder mehreren zusätzlichen flüchtigen organischen Verbindungen ausgewählt sind aus:
i) Butansäure, 1-Methylethylester;
ii) Pentansäure, 1-Methylethylester;
iii) p-Xylol;
iv) Propansäure, 1-Methylethylester; oder
v) Dimenthol;
und wobei eine Zunahme der Werte von i) bis iv) in Bezug auf ihre jeweiligen Kontrollwerte und eine Abnahme der Menge von v) in Bezug auf ihren Kontrollwert indikativ für eine erhöhte Wahrscheinlichkeit ist, dass das Individuum Dickdarmkrebs hat.

7. Verfahren nach Anspruch 4, wobei die Anwesenheit und Menge von Isopropanol und
alle in Anspruch 4 aufgeführten flüchtigen organischen Verbindungen bestimmt und ihre Mengen mit den jeweiligen Kontrollwerten verglichen werden.

8. Verfahren nach einem der vorherigen Ansprüche, wobei das Vorhandensein und die Menge der flüchtigen organischen Verbindung unter Verwendung von Gaschromatographie-Massenspektrometrie (GC-MS) bestimmt wird.

9. Verfahren nach einem der vorherigen Ansprüche, wobei das Individuum ein oder mehrere Symptome hat, die mit Dickdarmkrebs assoziiert sind,
wobei optional die mit Dickdarmkrebs assoziierten Symptome ausgewählt sind aus rektalen Blutungen, Durchfall, Verstopfung, Bauchkrämpfen, Bauchschmerzen, Müdigkeit oder Gewichtsverlust.

10. Verfahren nach einem der vorherigen Ansprüche, wobei das Individuum einen vorbestimmten Risikofaktor aufweist, der mit Dickdarmkrebs assoziiert ist,
wobei der vorbestimmte Risikofaktor, der mit Dickdarmkrebs assoziiert ist, optional basierend auf einem oder mehreren der folgenden Faktoren bestimmt wird:
i) ein positiver Fäkaler okkulter Blut Test (FOBT);
ii) das Vorhandensein von kolorektalen Polypen;
iii) Eisenmangelanämie;
iv) eine Erkrankung, die eine radiologische Bildgebung des Darms erfordert;
v) abnorme Darmgewohnheiten des Individuums (z. B. Verstopfung und/oder Durchfall);
vi) eine familiäre Vorbelastung mit kolorektalen Polypen; oder
vii) das Vorhandensein eines Polyposis-Syndroms.

11. Verfahren nach einem der Ansprüche 1 bis 10, wobei das Verfahren ein Bestimmen der Wahrscheinlichkeit involviert, dass ein Individuum ein kolorektales Adenokarzinom hat.

12. Verwendung von Isopropanol in einer Stuhlprobe als Biomarker für Dickdarmkrebs.

13. Verwendung nach Anspruch 12, wobei Isopropanol und eine oder mehrere zusätzliche flüchtige organische Verbindungen in einer Stuhlprobe als Biomarker für Dickdarmkrebs, wobei die eine oder mehreren zusätzlichen flüchtigen organischen Verbindungen ausgewählt sind aus:
i) 2-Hexanon;
ii) Butansäure, 3-Methyl-, Ethylester,
iii) Butansäure, 1-Methylethylester;
iv) Pentansäure, 1-Methylethylester;
v) p-Xylol;
vi) Propansäure, 1-Methylethylester; oder
vii) Dimenthol.

14. Verwendung nach Anspruch 13, wobei Isopropanol, Butansäure, 3-Methyl-, Ethylester und optional eine oder mehrere andere in Anspruch 13 aufgeführte flüchtige organische Verbindung als Biomarker für Dickdarmkrebs verwendet werden.

15. Verwendung nach einem der Ansprüche 13 oder 14, wobei Isopropanol, 2-Hexanon, Butansäure, 3-Methyl-, Ethylester und optional eine oder mehrere andere in Anspruch 13 aufgeführte flüchtige organische Verbindung als Biomarker für Dickdarmkrebs verwendet werden.

## Revendications

1. Procédé de détermination de la probabilité qu'un individu ait un cancer colorectal, le procédé comprenant les étapes de :
i) analyse d'un échantillon de selles prélevé sur ledit individu pour détecter si le composé organique volatil isopropanol est présent ;
ii) si de l'isopropanol est présent à l'étape i), détermination de la quantité d'isopropanol présente et comparaison de la quantité détectée à une valeur témoin ;
dans lequel la présence d'une quantité accrue d'isopropanol dans les selles par rapport à la valeur témoin indique une probabilité accrue que l'individu ait un cancer colorectal.

2. Procédé selon la revendication 1, dans lequel l'étape i) comprend en outre l'analyse de l'échantillon de selles prélevé sur ledit individu pour détecter la présence d'un ou plusieurs composés organiques volatils supplémentaires et, si un ou plusieurs composés organiques volatils supplémentaires sont présents, l'étape ii) comprend en outre la détermination de la quantité de l'un ou plusieurs composés organiques volatils supplémentaires et la comparaison de la quantité détectée à une ou plusieurs valeurs témoins pour chacun de l'un ou plusieurs composés organiques volatils supplémentaires ;
dans lequel une augmentation ou une diminution du niveau desdits un ou plusieurs composés organiques volatils supplémentaires par rapport à la valeur témoin est indicative de la probabilité que l'individu ait un cancer colorectal.

3. Procédé selon la revendication 2, dans lequel l'un ou plusieurs composés organiques volatils supplémentaires sont des composés organiques avec :
(i) un poids moléculaire inférieur ou égal à 250 Da ; et/ou
(ii) un point d'ébullition inférieur ou égal à 250° C lorsqu'il est mesuré à une pression atmosphérique standard de 101,3 kPa (1 atmosphère).

4. Procédé selon l'une quelconque des revendications 2 ou 3, dans lequel l'un ou plusieurs composés organiques volatils supplémentaires sont choisis parmi :
i) 2-hexanone ;
ii) Ester éthylique, 3-méthyl de l'acide butanoïque ;
iii) Ester 1-méthyléthylique de l'acide butanoïque,
iv) Ester 1-méthyléthylique de l'acide pentanoïque ;
v) p-Xylène ;
vi) Ester 1-méthyléthylique de l'acide propanoïque ; ou
vii) Dimenthol.

5. Procédé selon l'une quelconque des revendications précédentes, dans lequel ledit procédé comprend les étapes de :
i) analyse d'un échantillon de selles prélevé sur ledit individu pour détecter si les composés organiques volatils, isopropanol et ester éthylique, 3-méthyl- de l'acide butanoïque, sont présents ; et
ii) si de l'isopropanol et de l'ester éthylique, 3-méthyl- de l'acide butanoïque sont présents, la détermination de la quantité d'isopropanol et d'ester éthylique, 3-méthyl- de l'acide butanoïque présents et la comparaison de ces quantités à des valeurs témoins pour chaque composé organique volatil respectif ;
dans lequel une quantité accrue d'isopropanol et d'ester éthylique, 3-méthyl- de l'acide butanoïque par rapport à leurs valeurs témoins respectives indique une probabilité accrue que l'individu ait un cancer colorectal ;
éventuellement dans lequel l'étape i) comprend en outre l'analyse de l'échantillon de selles prélevé sur ledit individu pour détecter la présence d'un ou plusieurs composés organiques volatils supplémentaires et, si un ou plusieurs composés organiques volatils supplémentaires sont présents, l'étape ii) comprend en outre la détermination de la quantité de l'un ou plusieurs composés organiques volatils supplémentaires et la comparaison de cette quantité à une ou plusieurs valeurs témoins pour chacun de l'un ou plusieurs composés organiques volatils supplémentaires ;
dans lequel une augmentation ou une diminution du niveau desdits un ou plusieurs composés organiques volatils supplémentaires par rapport à la valeur témoin est indicative de la probabilité que l'individu ait un cancer colorectal ;
en outre éventuellement dans lequel l'un ou plusieurs composés organiques volatils supplémentaires sont choisis parmi
i) 2-hexanone ;
ii) Ester 1-méthyléthylique de l'acide butanoïque ;
iii) Ester 1-méthyléthylique de l'acide pentanoïque ;
iv) p-Xylène ;
v) Ester 1-méthyléthylique de l'acide propanoïque ; ou
vi) Dimenthol ;
et dans lequel une augmentation des niveaux de i) à v) par rapport à leurs valeurs témoins respectives et une diminution de la quantité de vi) par rapport à sa valeur témoin est indicative d'une probabilité accrue que l'individu ait un cancer colorectal.

6. Procédé selon l'une quelconque des revendications précédentes, dans lequel ledit procédé comprend les étapes de :
i) analyse de l'échantillon de selles prélevé sur ledit individu pour détecter si les composés organiques volatils, isopropanol, 2-hexanone et ester éthylique, 3-méthyl- de l'acide butanoïque sont présents ;
ii) si l'isopropanol, le 2-hexanone et l'ester éthylique, 3-méthyl- de l'acide butanoïque sont présents à l'étape i), détermination de la quantité d'isopropanol, de 2-hexanone et d'ester éthylique, 3-méthyl- de l'acide butanoïque présents et comparaison de cette quantité à des valeurs témoins pour chaque composé organique volatil respectif ;
dans lequel une quantité accrue d'isopropanol, de 2-hexanone et d'ester éthylique, 3-méthyl- de l'acide butanoïque par rapport à leurs valeurs témoins respectives indique une probabilité accrue que l'individu ait un cancer colorectal ;
éventuellement dans lequel l'étape i) comprend en outre l'analyse de l'échantillon de selles prélevé sur ledit individu pour détecter la présence d'un ou plusieurs composés organiques volatils supplémentaires et, si un ou plusieurs composés organiques volatils supplémentaires sont présents, l'étape ii) comprend en outre la détermination de la quantité de l'un ou plusieurs composés organiques volatils supplémentaires et la comparaison de cette quantité à une ou plusieurs valeurs témoins pour chacun de l'un ou plusieurs composés organiques volatils supplémentaires
dans lequel une augmentation ou une diminution du niveau desdits un ou plusieurs composés organiques volatils supplémentaires par rapport à la valeur témoin est indicative de la probabilité que l'individu ait un cancer colorectal ;
en outre éventuellement dans lequel l'un ou plusieurs composés organiques volatils supplémentaires sont choisis parmi :
i) Ester 1-méthyléthylique de l'acide butanoïque ;
ii) Ester 1-méthyléthylique de l'acide pentanoïque;
iii) p-Xylène ;
iv) Ester 1-méthyléthylique de l'acide propanoïque ; ou
v) Dimenthol ;
et dans lequel une augmentation des niveaux de i) à iv) par rapport à leurs valeurs témoins respectives et une diminution de la quantité de v) par rapport à sa valeur témoin est indicative d'une probabilité accrue que l'individu ait un cancer colorectal.

7. Procédé selon la revendication 4, dans lequel la présence et la quantité d'isopropanol et de tous les composés organiques volatils énumérés dans la revendication 4 sont déterminées, et les quantités de chacun sont comparées aux valeurs témoins respectives.

8. Procédé selon l'une quelconque des revendications précédentes, dans lequel la présence et la quantité du composé organique volatil sont déterminées en utilisant la spectrométrie de masse par chromatographie en phase gazeuse (GC-MS).

9. Procédé selon l'une quelconque des revendications précédentes, dans lequel l'individu présente un ou plusieurs symptômes associés au cancer colorectal ;
éventuellement dans lequel les symptômes associés au cancer colorectal sont choisis parmi les saignements rectaux, la diarrhée, la constipation, les crampes abdominales, les douleurs abdominales, la fatigue ou la perte de poids.

10. Procédé selon l'une quelconque des revendications précédentes, dans lequel l'individu présente un facteur de risque prédéterminé associé au cancer colorectal,
éventuellement dans lequel le facteur de risque prédéterminé associé au cancer colorectal est déterminé sur la base d'un ou plusieurs des éléments suivants :
i) un test de sang occulte fécal (FOBT) positif ;
ii) la présence de polypes colorectaux ;
iii) anémie ferriprive ;
iv) une affection nécessitant une imagerie radiologique de l'intestin ;
v) habitudes intestinales anormales de l'individu (par exemple, constipation et/ou diarrhée) ;
vi) des antécédents familiaux de polypes colorectaux ; ou
vii) la présence d'un syndrome de polypose.

11. Procédé selon l'une quelconque des revendications 1 à 10, dans lequel ledit procédé implique la détermination de la probabilité qu'un individu ait un adénocarcinome colorectal.

12. Utilisation de l'isopropanol dans un échantillon de selles comme biomarqueur du cancer colorectal.

13. Utilisation selon la revendication 12, dans laquelle l'isopropanol et un ou plusieurs composés organiques volatils supplémentaires dans un échantillon de selles en tant que biomarqueurs pour le cancer colorectal, dans laquelle l'un ou plusieurs composés organiques volatils supplémentaires sont choisis parmi :
i) 2-hexanone ;
ii) Ester éthylique, 3-méthyl- de l'acide butanoïque ;
iii) Ester 1-méthyléthylique de l'acide butanoïque ;
iv) Ester 1-méthyléthylique de l'acide pentanoïque ;
v) p-Xylène ;
vi) Ester 1-méthyléthylique de l'acide propanoïque; ou
vii) dimenthol.

14. Utilisation selon la revendication 13, dans laquelle l'isopropanol, l'ester éthylique, 3-méthyl- de l'acide butanoïque et éventuellement un ou plusieurs autres composés organiques volatils énumérés dans la revendication 13 sont utilisés comme biomarqueurs pour le cancer colorectal.

15. Utilisation selon l'une quelconque des revendications 13 ou 14, dans laquelle l'isopropanol, le 2-hexanone, l'ester éthylique, 3-méthyl- de l'acide butanoïque et éventuellement un ou plusieurs autres composés organiques volatils énumérés dans la revendication 13 sont utilisés comme biomarqueurs du cancer colorectal.
